(19) Europäisches Patentamt
European Patent Office
Office européen des brevets

(11) **EP 2 924 126 B1**

(12) **EUROPEAN PATENT SPECIFICATION**

(45) Date of publication and mention
of the grant of the patent:
**18.04.2018 Bulletin 2018/16**

(51) Int Cl.:
***C12Q 1/68*** (2018.01)

(21) Application number: **14162228.2**

(22) Date of filing: **28.03.2014**

(54) **Method for using microRNA (miRNA) for detection of endometriosis**

Verfahren zur Verwendung von microRNA (miRNA) zum Nachweis von Endometriose

Procédé d'utilisation de micro-ARN (miARN) pour la détection de l'endométriose

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**30.09.2015 Bulletin 2015/40**

(73) Proprietor: **Tervisetehnoloogiate Arenduskeskus AS**
**50410 Tartu (EE)**

(72) Inventors:
• **Saare, Merli**
**50410 Tartu (EE)**
• **Rekker, Kadri**
**50410 Tartu (EE)**
• **Sõritsa, Deniss**
**50410 Tartu (EE)**
• **Sõritsa, Andrei**
**50407 Tartu (EE)**
• **Salumets, Andres**
**50410 Tartu (EE)**
• **Peters, Maire**
**50410 Tartu (EE)**

(74) Representative: **Sarap, Margus**
**Sarap and Putk Patent Agency**
**Kompanii 1C**
**51004 Tartu (EE)**

(56) References cited:
• **E. MARIA C. OHLSSON TEAGUE ET AL: "MicroRNA-Regulated Pathways Associated with Endometriosis", MOLECULAR ENDOCRINOLOGY, vol. 23, no. 2, 1 February 2009 (2009-02-01), pages 265-275, XP055127849, ISSN: 0888-8809, DOI: 10.1210/me.2008-0387**
• **Y. RUAN: "Study on microRNA expression in endometrium of luteal phase and its relationship with infertility of endometriosis", ZHONGHUA FU CHAN KE ZA ZHI., vol. 48, no. 12, 1 December 2013 (2013-12-01), pages 907-910, XP055127853,**
• **WU R ET AL: "Identification of Differentially Expressed miRNAs in Ovarian Cancer from Endometriosis in the Same Patient", MODERN PATHOLOGY, vol. 27, no. Suppl. 2, February 2014 (2014-02), pages 467A-468A, XP002726874, & 103RD ANNUAL MEETING OF THE UNITED-STATES-AND-CANADIAN-ACADEMY-OF-PATHOLOGY (USCAP); SAN DIEGO, CA, USA; MARCH 01 -07, 2014**
• **WEN-TAO WANG ET AL: "Circulating MicroRNAs Identified in a Genome-Wide Serum MicroRNA Expression Analysis as Noninvasive Biomarkers for Endometriosis", JOURNAL OF CLINICAL ENDOCRINOLOGY & METABOLISM, vol. 98, no. 1, 1 January 2013 (2013-01-01), pages 281-289, XP055127854, ISSN: 0021-972X, DOI: 10.1210/jc.2012-2415**
• **S.-Z. JIA ET AL: "Plasma miR-17-5p, miR-20a and miR-22 are down-regulated in women with endometriosis", HUMAN REPRODUCTION, vol. 28, no. 2, 1 February 2013 (2013-02-01), pages 322-330, XP055127856, ISSN: 0268-1161, DOI: 10.1093/humrep/des413**

**Description**

**Field of the invention**

The present invention relates to the fields of molecular biology and medicine. More specifically the invention concerns the methods for investigation of endometriotic lesion specific miRNA expression to reveal signature of miRNAs that can be used as an alternative diagnostic tool for the discrimination of true endometriotic tissue samples from the lesion-like tissue samples.

**Background of the invention**

Endometriosis is a frequent gynecological disease which affects approximately 3% to 10% of women in the reproductive age, but the prevalence is much higher (40% to 80%) among women complaining of infertility or affected by pelvic pain. Endometriosis is characterized by the presence of endometrium-like tissue, consisting of both glandular epithelium and stroma, outside the uterus. The most common locations of endometriotic lesions are pelvic peritoneum, ovaries and rectovaginal region.

Regardless of the extensive studies, only a slight progress has been made in the development of non-invasive or tissue-based diagnostic tests. The gold standard for accurate diagnosis of endometriosis is still laparoscopy together with histological assessment of biopsied samples. Although histological evaluation of biopsied samples is routinely used in everyday practise, a number of studies have demonstrated that approximately 30-50% of surgical specimens removed during the laparoscopy are not confirmed during the histological assessments (Fernando *et al.,* 2013; Stegmann *et al.,* 2008).

Histologically, endometriotic lesions typically consist of endometrial glands surrounded by stromal cells. Usually inflammatory cells, such as hemosiderin-laden macrophages, may be present and in addition, the biopsied tissue samples include a various proportion of surrounding non-endometriotic cells of connective or fibrotic tissue. The amount of endometriotic cells in the biopsied samples may differ considerably and several histological sections may be necessary before identifying the glandular and stromal component characteristic to endometriosis. However, several studies have shown that laparoscopic visualisation of endometriotic lesions correlates with histopathologic diagnosis only in about half of the evaluated specimens (Marchino *et al.,* 2005; Wanyonyi *et al.,* 2011). The most common histopathologic findings of nonendometriotic lesions were fibrosis, hemorrhagic changes, inflammatory changes and normal peritoneal tissue or ovarian stroma (Wanyonyi *et al.,* 2011). Therefore, in some cases, endometriotic lesions may be difficult to recognise by routine hematoxylin-eosin staining.

miRNAs are believed to have a great potential to become a new frontier in molecular diagnostics. miRNAs are small (typically 22 nt in size) non-coding regulatory RNA molecules, which modulate the activity of specific mRNA targets. Different tissues/cell-types have their unique miRNA expression profiles (Gilabert-Estelles *et al.,* 2012; McCall *et al.,* 2011); therefore changes in miRNA expression that affect target mRNA degradation and/or translation may cause alterations in the firmly balanced regulation between miRNAs and their target mRNAs, and lead to pathological changes. An altered miRNA expression profiles of eutopic and/or ectopic endometrium from women with endometriosis have been described (Burney *et al.,* 2009; Laudanski *et al.,* 2013; Ramon *et al.,* 2011) suggesting the involvement of miRNAs in endometriosis pathogenesis. However, most of these studies have compared the miRNA profile of ectopic endometrium to eutopic endometrium of patients with or without endometriosis. As mentioned above, endometriotic lesions' biopsies usually contain only a small proportion of endometrial glands and stroma and a larger proportion of surrounding tissue with its own miRNA expression pattern, which may mask the disease-specific miRNA expression profile.

There is an urgent need for objective and reliable molecular diagnostic tests that allow comprehensive examination of biopsied samples to confirm the diagnosis of endometriosis.

**Summary of the invention**

The invention is as disclosed in the appended claims. The present invention provides an alternative method and diagnostic tool by measuring the expression levels of miRNAs which are differentially expressed in endometriotic lesions for the discrimination of true endometriotic tissue samples from the lesion-like tissue samples. The miRNA signature of this invention enables the evaluation of entire biopsied endometriotic tissue sample at the same time enabling diagnosing with high sensitivity and specificity. Accordingly, the invention comprises of method detecting whether the endometriotic lesions removed during the laparoscopic surgery contain cells characteristic to endometrial tissue. This molecular diagnostic method for discrimination of true endometriotic tissues samples from the lesion-like tissue samples measures the expression levels of miRNAs in endometriotic tissue samples relative to healthy control tissue. The miRNA signature in the present invention is determined for pelvic endometriotic lesions including peritoneal endometriotic lesions, rectovaginal endometriotic nodules, endometriomas of the ovary and endometriosis of uterus (adenomyosis). Specifically, the method of the present invention is based on a signature of biomarkers that comprises miRNA markers consisting of miR-200a-3p (SEQ ID NO: 1), miR-200b-3p (SEQ ID NO: 2), and miR-449a (SEQ ID NO: 3) where the higher expression levels can discriminate endometriotic lesions from the lesion-like tissue structures.

A method according to the present invention for identification an endometriotic lesion biopsy as true endometriotic lesion or non-diseased tissue comprises the steps of obtaining a sample of the endometriotic lesion tissue, extracting RNA of said endometriotic lesion sample and determining a miRNA expression profile of the endometriotic lesion sample and measuring the concentration of miR-200a-3p, miR-200b-3p,and, miR-449a, based on miRNA concentrations of non-diseased and diseased tissues determining the cut-off values discriminating non-diseased tissues from diseased tissues. Based on miRNA concentrations of the non-diseased and diseased tissues the cut-off values discriminating non-diseased tissues from diseased tissues are determined as follows: miR-200a>2.3, miR-200b>3.5, and miR-449>4.5.

## Detailed description of the Figures

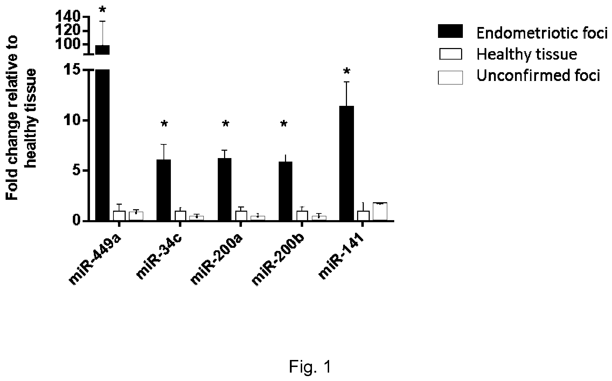

**Figure 1.** The expression of selected miRNAs in endometriotic lesions (n=22) and histologically unconfirmed lesion (n=10) groups using qRT-PCR. The fold changes for endometriotic lesions and unconfirmed lesion are calculated relative to healthy tissues (n=14) using the $2^{-\Delta\Delta CT}$ method (Livak *et al.,* 2001). The error bars denote SEM (standard error of the mean). Student's *t* test was used to evaluate expression differences of endometriotic lesions and unconfirmed lesions relative to healthy tissues. *denotes comparison of endometriotic lesions relative to healthy tissues, p<0.0001.

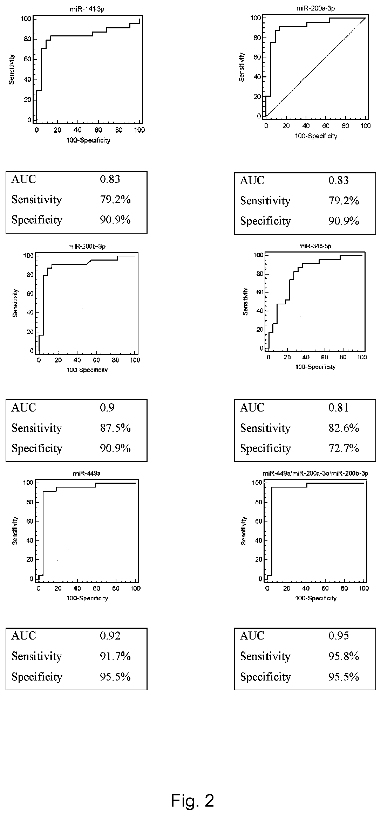

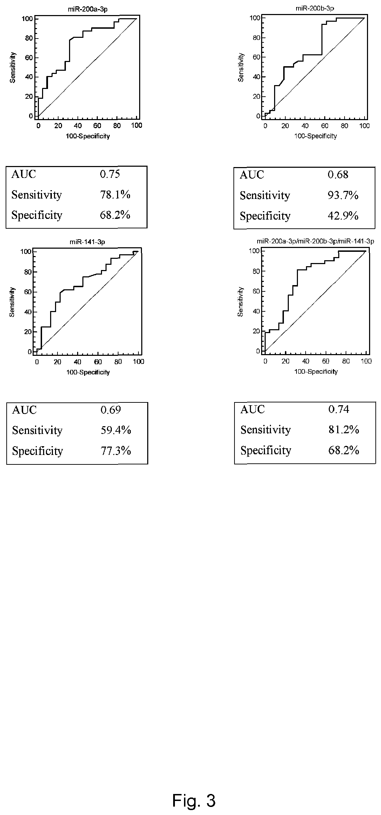

Figures 2-3. Receiver operating characteristic (ROC) curves for discriminating endometriotic lesions (n=22) from non-diseased tissues (n=24). and ROC curves for discriminating endometriosis patients (n=32) from disease-free women (n=22).

## Detailed description of the invention

As used herein the "sample" or "biological sample" is any biological material obtained from an individual. For example, it may be a endometriotic tissue biopsy obtained during laparoscopic operation.

The terms "microRNA" and "miRNA" and "miR"used herein can be used interchangeably.

The names of miRNAs, miR-200a-3p, miR-200b-3p, miR-449a and miR-200a, miR-200b, miR-449a and (SEQ ID 1, 2, 4) used herein can be used interchangeably.

The identification of full miRNome of endometriotic lesions is an important step towards developing novel miRNA-based non-invasive or tissue-based diagnostic tests for endometriosis diagnosis. In the present invention, a high-throughput sequencing to explore the endometriotic lesions specific full miRNA expression profile by comparing a set of paired samples of peritoneal endometriotic lesions and matched healthy surrounding tissues of the same endometriosis patients was conducted (Example 1). Data analysis revealed 3 miRNAs (SEQ ID 1,2,4) reliably distinguishing endometriotic lesions from lesion-like tissue structures

(Table 1).

| Table 1.List of experimental miRNAs. | | | |
|---|---|---|---|
| SEQ ID NO | ID | ACCESSION NUMBER (miRBase) | SEQUENCE |
| 1 | hsa-miR-200a-3p | MIMAT0000682 | UAACACUGUCUGGUAACGAUG |
| 2 | hsa-miR-200b-3p | MIMAT0000318 | UAAUACUGCCUGGUAAUGAUGA |
| 4 | hsa-miR-449a | MIMAT0001541 | UGGCAGUGUAUUGUUAGCUGGU |
| 8 | RNU44* | NR_002750 | CCTGGATGATGATAGCAAATGCTGACTGA ACATGAAGGTCTTAATTAGCTCTAACTGACT |

(continued)

| Table 1.List of experimental miRNAs. | | | |
|---|---|---|---|
| SEQ ID NO | ID | ACCESSION NUMBER (miRBase) | SEQUENCE |
| 9 | RNU48* | NR_002745 | GATGACCCCAGGTAACTCTGAGTGTGTCGC TGATGCCATCACCGCAGCGCTCTGACC |
| * small nucleolar RNA, NCBI Accession number | | | |

The miRNA expression levels cut-off values for miR-449a, miR-200a-3p, and miR-200b-3p that discriminate diseased tissues from non-diseased tissues were found. The microRNA-based Clinical Score Formula (CSF) was developed that enables to distinguish diseased tissues from non-diseased tissues according to the signature of three most discriminative miRNAs.

Three miRNAs, miR-449a, miR-200a-3p, and miR-200b-3p, showing different expression profile between endometriotic lesions and healthy tissues according to the sequencing data were selected for experimental validation. The expression of these three miRNAs was validated in 22 diseased and 24 non-diseased tissue samples using TaqMan qRT-PCR (Example 2). Non-diseased tissue samples were divided into two groups - adjacent healthy tissues (n = 14) and seemingly endometriotic lesions that upon subsequent histologic examination showed no evidence of endometriosis (n = 10) (Table 2).

Table 2. Clinical characteristics of patients and analyzed tissue samples used in the current study

| Patient ID | Age | BMI | Endometriosis Stage | Analysed tissue |
|---|---|---|---|---|
| E47* | 27 | 23 | III-IV | 47.1 Endometrium |
| | | | | 47.3 Cavum Douglas lesion |
| | | | | 47.4 Cavum Douglas healthy tissue |
| | | | | 47.5 Lig. latum dex. lesion |
| | | | | 47.6 Lig. latum dex. healthy tissue |
| | | | | 47.8 Lig.sacrouterina sin. lesion |
| | | | | 47.9 Lig.sacrouterina sin. healthy tissue |
| E101* | 28 | 22 | III-IV | 101.1 Endometrium |
| | | | | 101.2 Lig.sacrouterina dex. lesion |
| | | | | 101.3 Lig.sacrouterina dex. healthy tissue |
| | | | | 101.4 Lig.sacrouterina sin. lesion |
| E2 | 35 | 23 | II | E2.3 Lig.sacrouterina dex. lesion |
| E10 | 32 | | IV | E110.2 Uteral superficial lesion (non-diseased sample)** |
| E11 | 36 | 21 | III | E1 11.2 Lig.sacrouterina dex. lesion (non-diseased sample)** |
| | | | | E1 11.3 Fossa ovarica lesion (non-diseased sample)** |
| E15 | 42 | 18 | III | E15.2 Lig.sacrouterina sin.lesion |
| | | | | E15.4 Uteral superficial lesion (non-diseased sample)** |
| E16 | 37 | 26 | III | E16.4 Lig.sacrouterina sin.lesion |
| | | | | E16.6 Peritoneal lesion Cavum Douglas |

(continued)

| Patient ID | Age | BMI | Endometriosis Stage | Analysed tissue |
|---|---|---|---|---|
| E17 | 30 | 19 | III | E17.6 Lig.sacrouterina dex. lesion (non-diseased sample)** |
| | | | | E17.7 Uteral superficial lesion (non-diseased sample)** |
| E18 | 31 | 20 | III | E18.2 Lig.sacrouterina sin.lesion |
| | | | | E18.3 Uteral superficial lesion |
| E20 | 41 | 26 | III | E20.4 Uteral superficial lesion (non-diseased sample) |
| E40 | 33 | 22 | III-IV | E40.2 Lig. sacrouterina lesion |
| | | | | E40.4 Lig.sacrouterina healthy tissue |
| E41 | 39 | 25 | I-II | E41.2 Lig.sacrouterina dex. lesion |
| | | | | E41.4 Lig.sacrouterina dex. healthy tissue |
| E52 | 30 | 20 | II | E52.4 Lig.sacrouterina sin.lesion |
| E81 | 33 | 19 | I-II | 81.2 Lig.sacrouterina lesion |
| | | | | 81.3 Lig.sacrouterina healthy tissue |
| E100 | 39 | 27 | I-II | 100.2 Lig.sacrouterina sin.lesion |
| | | | | 100.4 Lig.sacrouterina dex. healthy tissue |
| E101 | 28 | 22 | III-IV | 101.5 Lig.sacrouterina sin. healthy tissue |
| E104 | 34 | 21 | I-II | 104.2 Fossa ovarica dex. |
| | | | | 104.3 Fossa ovarica dex. healthy tissue |
| | | | | 104.4 Lig. sacrouterina sin. |
| | | | | 104.5 Lig. sacrouterina sin. healthy tissue |
| E112 | 27 | 23 | I-II | 112.2 Lig.sacrouterina sin.lesion |
| | | | | 112.3 Lig.sacrouterina dex. healthy tissue |
| E123 | 36 | 21 | III | E123.2 Lig.sacrouterina sin.lesion (non-diseased sample)** |
| E138 | 29 | 23 | III | E138.2 Peritoneal lesion Cavum Douglas |
| E142 | 31 | 20 | IV | E142.3 Fossa ovarica lesion (non-diseased sample)** |
| | | | | E142.5 Uteral superficial lesion (non-diseased sample)** |
| E146 | 27 | 26 | - | E146.2 Lig.sacrouterina dex. healthy tissue |
| E147 | 39 | 29 | I | 147.3 Lig. sacrouterina sin.lesion |
| | | | | 147.4 Lig.sacrouterina dex. healthy tissue |
| E148 | 20 | 24 | III | 148.2 Lig.sacrouterina lesion |

* Samples used in sequencing study
**Seemingly endometriotic lesions that were not confirmed by histological analysis

The results of qRT-PCR indicated that adjacent healthy tissues and histologically unconfirmed lesion-like tissue samples showed very similar expression level of the tested miRNAs (all $p > 0.05$, Figure 1). All studied miRNAs showed significantly higher expression in endometriotic lesions (FC miR-449a = 95.7, miR-200a = 6.0, miR-200b = 5.69) compared to healthy tissue samples (all $p < 0.0001$) (Figure 1) (Example 3). When expression data of healthy tissue samples and unconfirmed lesion-like tissues were combined as one group, even more significant differences were seen between lesions and non-diseased group (FC miR-449a = 129.9, miR-200a = 10.5, miR-200b = 10.2 p < 0.0001).

Next, we constructed ROC curves to evaluate the ability of these miRNAs to discriminate between endometriotic lesions and non-diseased tissues (Example 4). The analysis of ROC revealed that all miRNAs enabled a correct classification of diseased and non-diseased tissues (AUC for miR-200a-3p, miR-200b-3p, and miR-449a were 0.91, 0.90, and 0.92 , respectively). When the miR-200a cut-off value was set to the optimal point >2.3, specificity was 90.9% and sensitivity was 87.5%, for miR-200b-3p cut-off value was set >3.5, specificity 90.9% and sensitivity 87.5%, for miR-449a cut-off value was set >4.6, specificity 95.5% and sensitivity 91.7%. The combined signature of three miRNAs - miR-449a/miR-200a-3p/miR-200b-3p showed the discriminating power with the highest sensitivity (95.8%) and specificity (95.5%) (AUC = 0.95, CI 0.83 - 0.99) (Figure 2).

Based on the expression levels of three miRNAs SEQ ID NO: 1, 2, , showing the best discrimination power the CSF, classifying tissue biopsies to diseased or non-diseased status, was calculated (Example 5). According to the invention CSF score of each sample was calculated by the formula:

$$X \times \Delta\text{Ct of miR- SEQ ID NO 4} + Y \times \Delta\text{Ct of miR- SEQ ID NO 1} - Z \times \Delta\text{Ct of miR- SEQ ID NO 2} - W, \text{ where}$$

where the X,Y, Z, and W are coefficients determined by the classifier algorithm and ΔCt is a threshold value difference of target and reference miRNAs. Based on the classifier algorithm the cut-off was calculated and all samples were divided into diseased and non-diseased samples. The CSF enabled to distinguish endometriotic tissue samples from lesion like samples with very high classification accuracy (>95). Using this CSF it is not essential to measure miRNA expression levels of non-disesed tissue samples together with test sample. Once the "non-diseased" tissue sample miRNA levels have been established, these levels can provide a basis for comparison without the need to rerun a control sample with each assay.

**Example 1**

**Study samples and RNA sequencing**

Eleven tissue samples (two endometria, five peritoneal lesion and four matched adjacent normal-appearing tissues) from two patients with a histologically confirmed diagnosis of moderate-severe endometriosis (III-IV stage) undergoing laparoscopy were recruited into the first stage of the research.

Endometrial biopsy samples were collected using an endometrial suction catheter (Pipelle, Laboratoire CCD, France), endometriotic lesions and macroscopically healthy surrounding tissues adjacent to the lesions (referred hereafter as 'healthy tissue') were removed during laparoscopy. Tissue samples were immediately placed into RNAlater (Ambion, Inc., Austin, Texas, USA). After 24-hour incubation in RNAlater, tissues were stored at -80°C until use.

Total RNA together with miRNA enriched fraction was extracted using RNeasy MinElute Cleanup kit in combination with miRNeasy Mini kit (Qiagen, Hilden, Germany) according to the manufacturer's instructions ("Preparation of miRNA-Enriched Fractions Separate from Larger RNAs", appendix A). The quality of total RNA was assessed on RNA 6000 Nano Chip using Agilent 2100 Bioanalyzer and the presence of miRNAs in enriched samples with Agilent Small RNA chips (Agilent Technologies, Palo Alto, CA, USA). Small RNA library construction and miRNA sequencing were performed as described previously (Velthut-Meikas *et al.,* 2013).

The quality of raw miRNA sequencing data was assessed by FastQC program (http://www.bioinformatics.babraham.ac.uk/projects/fastqc/). Filtered sequencing reads were analysed with miRDeep2 software (Friedlander et al., 2012) designed to predict known and novel miRNAs from deep sequencing data. Default parameters were used in all analysis steps. Briefly, adapter sequences were trimmed from the raw reads. Reads with at least 18 nucleotides were retained and then aligned to the human genome (NCBI build 37, hg19). Sequencing reads that mapped more than five times to the genome were discarded from the dataset. The remaining reads were used for generating potential miRNA precursors. These sequences were aligned to miRBase release 18 (Kozomara *et al.,* 2011) allowing one mismatch to detect previously annotated miRNAs and their expression levels from deep sequencing data. The reads predicted as potential novel miRNAs by miRDeep2 were subjected to BLAST to remove the sequences corresponding to human non-coding RNAs such as rRNA, snRNA, tRNA. Novel miRNAs were identified by the following criteria: read count five or more and miRDeep score at least 2. Annotated miRNA read counts were normalised to the total read counts of all reads per sample (expressed as counts per million). To test differential miRNA expression between endometriotic lesion, healthy tissue surrounding the lesion and eutopic endometrium, expression data for known miRNAs produced by miRDeep2 was used as input for the program R (version 2.15.2) package edgeR (Robinson *et al.,* 2010) available in Bioconductor version 2.8. Differential miRNA expression analysis was performed by edgeR and miRNAs with < 5 raw counts in more than half of the samples were discarded. The p-values were adjusted for multiple testing using the Benjamini and Hochberg method. miRNAs with FDR-adjusted p-values < 0.1 were considered statistically significant.

## Example 2

### Validation

For validation an additional tissue samples from women undergoing laparoscopy were included: 1) histologically confirmed peritoneal endometriotic lesions (n=22) and 2) non-diseased tissues (n=24). Non-diseased tissue samples were divided into two groups - adjacent healthy tissues (n = 14) and seemingly endometriotic lesions that upon subsequent histologic examination showed no evidence of endometriosis (n = 10). The general characteristics of patients and the list of studied tissues are presented in Table 2.

For histological examination of endometriotic lesion, OCT (Leica, Germany) embedded tissue sections were cut (10 $\mu$m), mounted on standard microscope slides, stained with hematoxylin/eosin and evaluated histologically. The sectioning, examination and tissue collection for miRNA extraction was performed as follows: after the trimming, five to ten tissue sections were stained, examined and in case of negative finding the following 10-15 slices were collected into the microtube. The sectioning, examination and collection of the tissue was carried on until a positive finding (presence of endometrial epithelial and stromal cells) or until all the sample was sectioned through (histologically negative finding). In case of positive finding, the sectioning was stopped and the remaining biopsy was added to the tube containing previously collected tissue sections.

Three miRNAs: hsa-miR-449a (Applied Biosystems, Assay ID 001030), miR-200a-3p-3p (ID 000502), and miR-200b-3p-3p (ID 002251) were selected for validation analysis. Two small nucleolar RNAs, RNU44 (ID 001094) and RNU48 (ID 001006) were used as endogenous controls. cDNA synthesis was conducted from miRNA enriched RNA fraction with TaqMan® MicroRNA Reverse Transcription Kit (Applied Biosystems, California, US). Real-time PCR was performed in 7500 Fast Real-Time PCR System (Applied Biosystems) using TaqMan® Universal PCR Master Mix, No AmpErase® UNG (Applied Biosystems) and following the manufacturer's instructions.

For each individual sample, the mean threshold value Ct (the number of cycles required for the florescent signal to cross the minimal detection treshold) is calculated. To minimize errors and the effect of sample-to-sample variation, the normalization using two endogenous controls is performed and the threshold value difference ($\Delta$Ct) of target and reference miRNAs is calculated.

## Example 3

### Statistical tests

The $2^{-\Delta\Delta Ct}$ method (Livak *et al.,* 2001) was used for calculating the relative expression and to determine the fold change of miRNA expression between endometriotic lesion and adjacent healthy tissue. Normalized miRNA expression level differences between the endometriotic lesion and healthy tissues were analysed using Student's two sided t-test and p < 0.05 was considered as a statistically significant difference.

## Example 4

### ROC curve

Receiver-operating characteristic (ROC) curves, together with sensitivity and specificity were calculated using MedCalc software version 12.7.5 (www.medcalc.org). For the joint effect of the best miRNA signature, the multivariate logistic regression model was utilized as described earlier (Torres *et al.,* 2013).

## Example 5

### CSF formula

To find a CSF formula that classifies samples into healthy and endometriosis group based on the SEQ ID NO: 1, 2, and , miRNAs, linear soft margin Support Vector Machines (SVM) classifier was trained using package e1071 in statistics language and environment R (version 3.0.1). Regularization parameter C was chosen by performing a grid search to find the value with lowest 10-fold cross-validation error.

**References**

Burney RO, Hamilton AE, Aghajanova L, Vo KC, Nezhat CN, Lessey BA, Giudice LC. MicroRNA expression profiling of eutopic secretory endometrium in women with versus without endometriosis. Mol Hum Reprod 2009; 15; 10:625-631.

Fernando S, Soh PQ, Cooper M, Evans S, Reid G, Tsaltas J, Rombauts L. Reliability of visual diagnosis of endometriosis. JMinim Invasive Gynecol 2013; 20; 6:783-789.

Gilabert-Estelles J, Braza-Boils A, Ramon LA, Zorio E, Medina P, Espana F, Estelles A. Role of microRNAs in gynecological pathology. Curr Med Chem 2012; 19; 15:2406-2413.

Kozomara A, Griffiths-Jones S. miRBase: integrating microRNA annotation and deep-sequencing data. Nucleic Acids Res 2011; 39; Database issue:D152-157.

Laudanski P, Charkiewicz R, Kuzmicki M, Szamatowicz J, Charkiewicz A, Niklinski J. MicroRNAs expression profiling of eutopic proliferative endometrium in women with ovarian endometriosis. Reprod Biol Endocrinol 2013; 11; 1:78.

Livak KJ, Schmittgen TD. Analysis of relative gene expression data using real-time quantitative PCR and the 2(-Delta Delta C(T)) Method. Methods 2001; 25; 4:402-408.

Marchino GL, Gennarelli G, Enria R, Bongioanni F, Lipari G, Massobrio M. Diagnosis of pelvic endometriosis with use of macroscopic versus histologic findings. Fertil Steril 2005; 84; 1:12-15.

McCall MN, Kent OA, Yu J, Fox-Talbot K, Zaiman AL, Halushka MK. MicroRNA profiling of diverse endothelial cell types. BMC Med Genomics 2011; 4; 78.

Ramon LA, Braza-Boils A, Gilabert-Estelles J, Gilabert J, Espana F, Chirivella M, Estelles A. microRNAs expression in endometriosis and their relation to angiogenic factors. Hum Reprod 2011; 26; 5:1082-1090.

Robinson MD, McCarthy DJ, Smyth GK. edgeR: a Bioconductor package for differential expression analysis of digital gene expression data. Bioinformatics 2010; 26; 1:139-140.

Stegmann BJ, Sinaii N, Liu S, Segars J, Merino M, Nieman LK, Stratton P. Using location, color, size, and depth to characterize and identify endometriosis lesions in a cohort of 133 women. Fertil Steril 2008; 89; 6:1632-1636.

Torres A, Torres K, Pesci A, Ceccaroni M, Paszkowski T, Cassandrini P, Zamboni G, Maciejewski R. Diagnostic and prognostic significance of miRNA signatures in tissues and plasma of endometrioid endometrial carcinoma patients. Int J Cancer 2013; 132; 7:1633-1645.

Wanyonyi SZ, Sequeira E, Mukono SG. Correlation between laparoscopic and histopathologic diagnosis of endometriosis. Int J Gynaecol Obstet 2011; 115; 3:273-276.

Velthut-Meikas A, Simm J, Tuuri T, Tapanainen JS, Metsis M, Salumets A. Research Resource: Small RNA-seq of Human Granulosa Cells Reveals miRNAs in FSHR and Aromatase Genes. Mol Endocrinol 2013; 27; 7:1128-1141.

**Claims**

1. A method of detecting endometriosis by measuring the concentration of miRNAs in vitro comprising the steps of:

   extracting RNA from an endometriotic lesion tissue sample and determining a miRNA expression profile of the endometriotic lesion sample and measuring the concentration of miRNAs namely miR-200a-3p, miR-200b-3p and miR-449a, based on evaluating the expression level of said three miRNAs, discriminating an endometriotic lesion biopsy as true endometriotic lesion from non-diseased tissue wherein the increased expression of the miRNAs compared to a healthy sample is an indication of endometriosis.

2. The method according to claim 1 **characterised by** that the miRNA expression profile comprises expression profiles of miRNAs namely hsa-miR-200a-3p, hsa-miR-200b-3p and hsa-miR-449a and determined cut-off values of said three miRNAs are used for calculating the clinical score (CS) according to the clinical score formula I

$$X \times \Delta Ct \text{ of miRNA} + Y \times \Delta Ct \text{ of miRNA} - Z \times \Delta Ct \text{ of miRNA} - W,$$

where X, Y, Z, and W are coefficients determined by the classifier algorithm and ΔCt is a threshold value difference of target and reference miRNA and wherein the CS lower than 0 indicates that the sample of the endometriotic lesion tissue is a true endometriotic lesion, and the clinical score higher than 0 indicates that the sample of the endometriotic lesion tissue is disease-free.

3. The method according to claim 1 **characterised by** that the miRNA expression profile comprises the increased expression from expression profiles of miRNAs namely of miR-200a-3p (SEQ NO. 1), miR-200b-3p (SEQ NO. 2) and miR-449a (SEQ No. 4).

**Patentansprüche**

1. Verfahren zum Nachweis von Endometriose durch Konzentrationsmessung der miRNAs in vitro, bestehend aus folgenden Schritten:

Entnahme der RNA einer endometrisch geschädigten Gewebeprobe und Bestimmung eines miRNA-Expressionsprofils der geschädigten Gewebeprobe, sowie Konzentrationsmessung der miRNAs miR-200a-3p, miR-200b-3p und miR-449a, basierend auf der Auswertung des Expressionsniveaus der genannten drei miRNAs durch Unterscheidung einer endometrisch geschädigten Gewebeentnahme als tatsächliche endometrische Schädigung gegenüber nicht befallenem Gewebe, wobei die erhöhte Expression der miRNAs verglichen mit gesunden Proben ein Anzeichen für Endometriose ist.

2. Verfahren nach Anspruch 1, **dadurch gekennzeichnet, dass** das miRNA-Expressionsprofil aus Expressionsprofilen der miRNAs hsa-miR-200a-3p, hsa-miR-200b-3p und hsa-miR-449a besteht und die bestimmten Grenzwerte der genannten miRNAs zur Berechnung des klinischen Wertes (CS) nach Formel I

$$X \times \Delta Ct \text{ der miRNA} + Y \times \Delta Ct \text{ der miRNA} - Z \times \Delta Ct \text{ der miRNA} - W,$$

verwendet werden, wobei X, Y, Z, und W Koeffizienten sind, die durch den Klassifizierungsalgorithmus bestimmt sind und ΔCt eine Zielgrenzwertdifferenz der Ziel- und Bezugs-miRNA ist, und wobei ein CS-Wert unter 0 anzeigt, dass die Probe des endometrisch geschädigten Gewebes eine tatsächliche, endometrische Schädigung darstellt, und wobei ein klinischer Wert über 0 anzeigt, dass die endometrisch geschädigte Gewebeprobe nicht befallen ist.

3. Verfahren nach Anspruch 1, **dadurch gekennzeichnet, dass** das miRNA-Expressionsprofil die erhöhte Expression der Expressionsprofile der miRNAs miR-200-3p (SEQ NO. 1), miR-200b-3p (SEQ NO.2) und miR-449a (SEQ NO. 4) umfasst.

**Revendications**

1. Procédé de détection de l'endométriose en mesurant la concentration de miARNs in vitro comprenant les étapes de:

extraire l'ARN d'un échantillon de tissu de lésion endométriosique et déterminer un profil d'expression de miARN de l'échantillon de lésion endométriosique et mesurer la concentration de miARN, à savoir miR-200a-3p, miR-200b-3p et miR-449a, basé sur l'évaluation du niveau d'expression des trois miARNs précités discriminant une biopsie de lésion endométriosique en tant que véritable lésion endométriotique d'un tissu non-malade dans lequel l'expression accrue des miARN par rapport à un échantillon sain est une indication de l'endométriose.

2. Procédé selon la revendication 1 **caractérisée en ce que** le profil d'expression de miRNA comprend des profils d'expression de miARN à savoir hsa-miR-200a-3p, hsa-miR-200b-3p et hsa-miR-449a et des valeurs de coupure déterminées des trois miARN précités sont utilisés pour calculer le score clinique (CS) selon la formule du score clinique I

$$X \times \Delta Ct \text{ of } miRNA + Y \times \Delta Ct \text{ of } miRNA - Z \times \Delta Ct \text{ of } miRNA - W,$$

où X, Y, Z et W sont des coefficients déterminés par l'algorithme classificateur et Ct est une différence de valeur de seuil de miRNA cible et de référence et où le CS inférieur à 0 indique que l'échantillon du tissu de lésion endométriosique est une véritable lésion endométriosique, et le score clinique supérieur à 0 indique que l'échantillon du tissu lésion endométriosique est indemne.

3. Procédé selon la revendication 1, **caractérisé en ce que** le profil d'expression de miARN comprend l'expression accrue de profils d'expression de miARN à savoir de miR-200a-3p (SEQ N ° 1), miR-200b-3p (SEQ N ° 2) et miR-449a (SEQ n ° 4).

Fig. 1

Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Mol Hum Reprod,* 2009, vol. 15 (10), 625-631
- **FERNANDO S ; SOH PQ ; COOPER M ; EVANS S ; REID G ; TSALTAS J ; ROMBAUTS L.** Reliability of visual diagnosis of endometriosis. *JMinim Invasive Gynecol,* 2013, vol. 20 (6), 783-789
- **GILABERT-ESTELLES J ; BRAZA-BOILS A ; RAMON LA ; ZORIO E ; MEDINA P ; ESPANA F ; ESTELLES A.** Role of microRNAs in gynecological pathology. *Curr Med Chem,* 2012, vol. 19 (15), 2406-2413
- **KOZOMARA A ; GRIFFITHS-JONES S.** miRBase: integrating microRNA annotation and deep-sequencing data. *Nucleic Acids Res,* 2011, vol. 39, D152-157
- **LAUDANSKI P ; CHARKIEWICZ R ; KUZMICKI M ; SZAMATOWICZ J ; CHARKIEWICZ A ; NIKLINSKI J.** MicroRNAs expression profiling of eutopic proliferative endometrium in women with ovarian endometriosis. *Reprod Biol Endocrinol,* 2013, vol. 11 (1), 78
- **LIVAK KJ ; SCHMITTGEN TD.** Analysis of relative gene expression data using real-time quantitative PCR and the 2(-Delta Delta C(T)) Method. *Methods,* 2001, vol. 25 (4), 402-408
- **MARCHINO GL ; GENNARELLI G ; ENRIA R ; BONGIOANNI F ; LIPARI G ; MASSOBRIO M.** Diagnosis of pelvic endometriosis with use of macroscopic versus histologic findings. *Fertil Steril,* 2005, vol. 84 (1), 12-15
- **MCCALL MN ; KENT OA ; YU J ; FOX-TALBOT K ; ZAIMAN AL ; HALUSHKA MK.** MicroRNA profiling of diverse endothelial cell types. *BMC Med Genomics,* 2011, vol. 4, 78
- **RAMON LA ; BRAZA-BOILS A ; GILABERT-ESTELLES J ; GILABERT J ; ESPANA F ; CHIRIVELLA M ; ESTELLES A.** microRNAs expression in endometriosis and their relation to angiogenic factors. *Hum Reprod,* 2011, vol. 26 (5), 1082-1090
- **ROBINSON MD ; MCCARTHY DJ ; SMYTH GK.** edgeR: a Bioconductor package for differential expression analysis of digital gene expression data. *Bioinformatics,* 2010, vol. 26 (1), 139-140
- **STEGMANN BJ ; SINAII N ; LIU S ; SEGARS J ; MERINO M ; NIEMAN LK ; STRATTON P.** Using location, color, size, and depth to characterize and identify endometriosis lesions in a cohort of 133 women. *Fertil Steril,* 2008, vol. 89 (6), 1632-1636
- **TORRES A ; TORRES K ; PESCI A ; CECCARONI M ; PASZKOWSKI T ; CASSANDRINI P ; ZAMBONI G ; MACIEJEWSKI R.** Diagnostic and prognostic significance of miRNA signatures in tissues and plasma of endometrioid endometrial carcinoma patients. *Int J Cancer,* 2013, vol. 132 (7), 1633-1645
- **WANYONYI SZ ; SEQUEIRA E ; MUKONO SG.** Correlation between laparoscopic and histopathologic diagnosis of endometriosis. *Int J Gynaecol Obstet,* 2011, vol. 115 (3), 273-276
- **VELTHUT-MEIKAS A ; SIMM J ; TUURI T ; TAPANAINEN JS ; METSIS M ; SALUMETS A.** Research Resource: Small RNA-seq of Human Granulosa Cells Reveals miRNAs in FSHR and Aromatase Genes. *Mol Endocrinol,* 2013, vol. 27 (7), 1128-1141

SEQUENCE LISTING

<110> Reproduktiivmeditsiini TAK AS

<120> Method for using microRNA (miRNA) for detection of endometriosis

<130> 328/P1-EP

<160> 9

<170> BiSSAP 1.3

<210> 1
<211> 21
<212> RNA
<213> Artificial Sequence

<220>
<223> miRBase No. MIMAT0000682

<400> 1
uaacacuguc ugguaacgau g        21

<210> 2
<211> 22
<212> RNA
<213> Artificial Sequence

<220>
<223> miRBase No. MIMAT0000318

<400> 2
uaauacugcc ugguaaugau ga        22

<210> 3
<211> 22
<212> RNA
<213> Artificial Sequence

<220>
<223> miRBase No. MIMAT0000432

<400> 3
uaacacuguc ugguaaagau gg        22

<210> 4
<211> 22
<212> RNA
<213> Artificial Sequence

<220>
<223> miRBase No. MIMAT0001541

<400> 4
uggcagugua uuguuagcug gu        22

<210> 5
<211> 23
<212> RNA
<213> Artificial Sequence

<220>
<223> miRBase No. MIMAT0000686

<400> 5
aggcagugua guuagcugau ugc        23

<210> 6
<211> 22
<212> RNA
<213> Artificial Sequence

<220>
<223> miRBase No. MIMAT0000692

<400> 6
uguaaacauc cuugacugga ag        22

<210> 7
<211> 22
<212> RNA
<213> Artificial Sequence

<220>
<223> miRBase No. MIMAT0000097

<400> 7
aacccguaga uccgaucuug ug        22

<210> 8
<211> 60
<212> RNA
<213> Artificial Sequence

<220>
<223> NCBI No. 002750

<400> 8
cctggatgat gatagcaaat gctgactgaa catgaaggtc ttaattagct ctaactgact        60

<210> 9
<211> 57
<212> RNA
<213> Artificial Sequence

<220>
<223> NCBI No. 002745

<400> 9
gatgacccca ggtaactctg agtgtgtcgc tgatgccatc accgcagcgc tctgacc        57